# EUROPEAN PATENT APPLICATION

(11) **EP 3 808 375 A1**
(43) Date of publication of application: **21.04.2021**
(21) Application number: 19811930.7
(22) Date of filing: 27.05.2019
(51) Int. Cl.: A61K 39/395, A61K 45/06, A61P 35/00, A61K 39/00

(54) **PHARMACEUTICAL COMPOSITION FOR PREVENTING OR TREATING CANCER, CONTAINING CD300C EXPRESSION INHIBITOR OR ACTIVITY INHIBITOR**

(30) Priority: 31.05.2018 KR 20180062620; 24.05.2019 KR 20190061067
(71) Applicant: CentricsBio, Inc., Seoul 05836 (KR)
(72) Inventor: JEON, Jae-Won, Seoul 07598 (KR); JUNG, Joon-Goo, Sejong-Si 30098 (KR)
(74) Representative: Dehns
(86) International application number: PCT/KR2019/006307
(87) International publication number: WO 2019/231188

(57) **Abstract**

The present invention relates to: a pharmaceutical composition for preventing or treating cancer, containing a CD300c expression inhibitor or activity inhibitor; and the like. A CD300c expression inhibitor or activity inhibitor, according to the present invention, in a cancer environment, increases the number of tumor-infiltrating lymphocytes and cytotoxic T cells, reduces the number of myeloid-derived suppressor cells and can effectively inhibit the growth and development of cancer, and thus is expected to be effectively usable as an immunotherapeutic agent in the treatment of various cancers.

## Description

### TECHNICAL FIELD

The present invention relates to a pharmaceutical composition including a CD300c protein expression inhibitor or activity inhibitor, and the like.

### BACKGROUND ART

Cancer is one of the diseases that account for the largest share of the causes of death in modern people, is a disease caused by changes in normal cells due to gene mutations occurring due to various causes, and refers to malignant tumors that do not follow normal cell differentiation, proliferation, growth patterns, and the like. Cancer is characterized by "uncontrolled cell growth," and this abnormal cell growth causes the formation of a mass of cells called a tumor, which infiltrates the surrounding tissues and, in severe cases, may metastasize to other organs of the body. Cancer is an intractable chronic disease that in many cases cannot be fundamentally cured even by surgery, radiotherapy, or chemotherapy, causes pain to patients, and ultimately leads to death.

Cancer drug treatments, that is, anticancer drugs are compounds that generally have cytotoxicity, and treat cancer by attacking and killing cancer cells, but exhibit high side effects since they damage not only cancer cells but also normal cells. Thus, in order to reduce side effects, target anticancer drugs have been developed. However, in the case of these target anticancer drugs, side effects can be reduced, but there is a limitation that resistance occurs with a high probability (Korean Patent Publication No. 10-2018-0099557). Therefore, in recent years, interest in immune anticancer drugs that reduce problems due to toxicity and resistance using the body's immune system tends to rapidly increase. As an example of such an immune anticancer agent, an immune checkpoint inhibitor that binds to PD-L1 on the surface of cancer cells and inhibits the binding of T cells to PD-1 to activate T cells and attack cancer cells has been developed. However, even these immune checkpoint inhibitors are not effective in various types of cancer, and therefore, there is a need to develop a novel immune checkpoint inhibitor that exhibits the same therapeutic effect in various cancers.

Thus, the inventors of the present invention had conducted intensive research on a protein that is expressed on the surface of cancer cells, such as PD-L1 and inhibits the expression of T cells in various cancers, and consequently completed the present invention.

### DESCRIPTION OF EMBODIMENTS

### TECHNICAL PROBLEM

The present invention was devised to solve the above-described problems, it has been confirmed that, by inhibiting the expression or activity of a CD300c protein presented on the surfaces of various cancer cells, the activity of T cells is increased, and the proliferation of cancer cells can be suppressed, and an object of the present invention is to provide a pharmaceutical composition for preventing or treating cancer, including, as an active ingredient, a CD300c expression inhibitor or activity inhibitor, and the like.

However, technical problems to be solved by the present invention are not limited to the above-described technical problems, and other unmentioned technical problems will become apparent from the following description to those of ordinary skill in the art.

### TECHNICAL SOLUTION

According to an aspect of the present invention, there is provided a pharmaceutical composition for preventing or treating cancer, including, as an active ingredient, a CD300c expression inhibitor or CD300c activity inhibitor.

In one embodiment of the present invention, the CD300c expression inhibitor is preferably an antisense oligonucleotide (ASO), small hairpin RNA, small interfering RNA (siRNA), a ribozyme, or the like that complementarily binds to mRNA of the CD300c gene, but is not limited as long as it is a material that reduces or inhibits the expression of the CD300c gene. The mechanism by which the material inhibits the expression of CD300c is not particularly limited, and, for example, may act as a mechanism for inhibiting gene expression such as transcription and translation.

In another embodiment of the present invention, the CD300c activity inhibitor is a compound, peptide, peptide mimetic, substrate analog, aptamer, antibody, or the like that complementarily binds to the CD300c protein, but is not limited as long as it is a material that reduces or inhibits the activity of CD300c by binding to the CD300c protein. The mechanism by which the material inhibits the activity of the CD300c protein is not particularly limited, and, for example, may act as a mechanism for converting an active form to an inactive form. In one embodiment, the antibody may be a polyclonal antibody or a monoclonal antibody, preferably a human monoclonal anti-CD300c antibody, or an antibody fragment, but is not limited as long as it is an antibody that specifically binds to CD300c. A soluble receptor is a receptor that binds to CD300c, preferably includes a sequence that specifically binds to the amino acid sequence of SEQ ID NO: 1, but is not limited thereto as long as it is a receptor that binds to CD300c.

In another embodiment of the present invention, the cancer is preferably colorectal cancer, rectal cancer, colon cancer, thyroid cancer, oral cancer, pharyngeal cancer, laryngeal cancer, cervical cancer, brain cancer, lung cancer, ovarian cancer, bladder cancer, kidney cancer, liver cancer, pancreatic cancer, prostate cancer, skin cancer, tongue cancer, breast cancer, uterine cancer, gastric cancer, bone cancer, blood cancer, or the like, but is not limited thereto as long as it is the type of cancer that expresses the CD300c protein on the surface of cancer cells.

In another embodiment of the present invention, the pharmaceutical composition may further include other existing anticancer agents, and the anticancer agents are preferably doxorubicin, cisplatin, gemcitabine, oxaliplatin, 5-FU, cetuximab, panitumumab, nimotuzumab, necitumumab, cancer antigens, anticancer viruses, and the like, but are not limited thereto as long as they are materials that are currently used as anticancer agents. The cancer antigens are cancer vaccines specific to carcinomas, preferably NY-ESO-1 as a bladder cancer-specific cancer antigen, HER2 as a breast cancer-specific cancer antigen, CEA as a colorectal cancer-specific cancer antigen, and VEGFR1 and VEGFR2 as lung cancer-specific cancer antigens, but are not limited thereto as long as they are types of cancer antigens known as cancer vaccines. Examples of anticancer viruses include Imlygic and Pexa-Vec, but are not limited thereto as long as they are known anticancer viruses. The anticancer agent may be further included preferably via coadministration, or may be in a form bound to the inhibitor of the present invention, or may be in a form included together in a carrier of the anticancer agent.

In another embodiment of the present invention, the pharmaceutical composition may inhibit the proliferation, survival, metastasis, and recurrence of cancer or cancer stem cells, and resistance to anticancer agents, but the effects are not limited thereto as long as they are effects obtained by the pharmaceutical composition of the present invention.

The present invention also provides a method of screening for a material for preventing or treating cancer, the method including: (a) culturing a cancer cell expressing a CD300c protein; (b) treating the cultured cancer cell with a candidate material; (c) measuring a CD300c expression level of the cell treated with the candidate material; and (d) selecting a candidate material that reduces the CD300c expression level.

The present invention also provides a method of screening for a material for preventing or treating cancer, the method including: (a) treating a CD300c protein with a candidate material; and (b) selecting a candidate material bound to the CD300c protein.

In one embodiment of the present invention, the measurement of the expression level refers to measurement of the expression level of mRNA and/or a protein, and the measurement of the expression level of mRNA refers to confirmation of whether CD300c mRNA is present in a biological sample and the expression level thereof, which can be confirmed by measuring the amount of mRNA. Analysis methods for this include RT-PCR, competitive RT-PCR, real-time RT-PCR, RNase protection assay (RPA), northern blotting, and DNA microarray chips, but the present invention is not limited thereto. In addition, the measurement of the expression level of a protein refers to confirmation of whether a CD300c protein is present in a biological sample and the expression level thereof, which can be confirmed by measuring the amount of the protein using an antibody specifically binding to the CD300c protein or measuring the activity of the protein. Analysis methods for this include western blotting, enzyme linked immunosorbent assay (ELISA), radioimmunoassay, radioimmunodiffusion, Ouchterlony immunodiffusion, Rocket immunoelectrophoresis, immunohistochemical staining, immunoprecipitation assay, complete fixation assay, FACS, and protein chips, but the present invention is not limited thereto.

In another embodiment of the present invention, the selection of the material bound to the candidate material is a method of selecting a material bound to the CD300c protein, and analysis methods for this include western blotting, enzyme linked immunosorbent assay (ELISA), radioimmunoassay, radioimmunodiffusion, Ouchterlony immunodiffusion, Rocket immunoelectrophoresis, immunohistochemical staining, immunoprecipitation assay, complete fixation assay, FACS, and protein chips, but the present invention is not limited thereto.

In another embodiment of the present invention, the candidate material is, but is not limited to, a nucleotide, DNA, RNA, an amino acid, an aptamer, a protein, a compound, a natural product, a natural extract, or a vector.

The present invention also provides a method of treating cancer, including administering, to an individual, a pharmaceutical composition including, as an active ingredient, a CD300c expression inhibitor or CD300c activity inhibitor.

The present invention also provides a use of a pharmaceutical composition for preventing or treating cancer, the pharmaceutical composition including, as an active ingredient, a CD300c expression inhibitor or CD300c activity inhibitor.

### ADVANTAGEOUS EFFECTS OF INVENTION

A CD300c expression inhibitor or activity inhibitor according to the present invention activates T cells by binding to CD300c expressed on the surface of various cancers or inhibiting the expression of CD300c and at the same time, inhibits the proliferation of cancer cells, and thus can be effectively used as an immunotherapeutic agent for various cancers. Such an inhibitor can increase the number of tumor-infiltrating lymphocytes and cytotoxic T lymphocytes in a cancer environment, reduce the number of myeloid-derived suppressor cells, and also effectively inhibit the growth and development of cancer, and thus the CD300c expression inhibitor or activity inhibitor of the present invention is expected to be effectively used in cancer treatment as a novel immunotherapeutic agent.

### BRIEF DESCRIPTION OF DRAWINGS

FIG. 1 is a view showing SDS-PAGE results confirming sCD300c-Fc according to an embodiment of the present invention.
FIG. 2 is a graph showing results confirming the effect of sCD300c-Fc according to an embodiment of the present invention on tumor-infiltrating lymphocytes.
FIG. 3 is a graph showing results confirming the effect of sCD300c-Fc according to an embodiment of the present invention on the signaling mechanism of NF-κB.
FIG. 4 is a graph showing results confirming the effect of an anti-CD300c antibody according to an embodiment of the present invention on human T cells.
FIG. 5 illustrates results confirming the inhibitory effect of an anti-CD300c antibody according to an embodiment of the present invention on lung cancer growth.
FIG. 6 is a graph showing results confirming the inhibitory effect of an anti-CD300c antibody according to an embodiment of the present invention on breast cancer growth.
FIG. 7 is a graph showing results confirming the inhibitory effect of an anti-CD300c antibody according to an embodiment of the present invention on colorectal cancer growth.
FIG. 8 is a graph showing results confirming the inhibitory effect of an anti-CD300c antibody according to an embodiment of the present invention on cancer growth *in vivo.*
FIG. 9 is a graph showing results confirming the inhibitory effect of an anti-CD300c antibody according to an embodiment of the present invention on cancer growth *in vivo.*
FIG. 10 illustrates results confirming the inhibitory effect of CD300c siRNA according to an embodiment of the present invention on cancer growth.
FIG. 11 illustrates results confirming the effect of an anti-CD300c antibody according to an embodiment of the present invention on an anticancer immune response.
FIG. 12 is a schematic view illustrating a mechanism exhibiting an anticancer effect by inhibiting the function and/or expression of CD300c.

### BEST MODE

A CD300c expression inhibitor or activity inhibitor of the present invention can increase the number of tumor-infiltrating lymphocytes and cytotoxic T lymphocytes in a cancer environment, reduce the number of myeloid-derived suppressor cells, and also effectively inhibit the growth and development of cancer, and thus may be effectively used in the treatment of various cancers that express CD300c on the surface.

In the present specification, "antibody" includes immunoglobulin molecules that are immunologically reactive with a specific antigen, and includes all of polyclonal antibodies, monoclonal antibodies, and functional fragments thereof. In addition, the term may include forms produced by genetic engineering, such as chimeric antibodies (e.g., humanized murine antibodies) and heterologous antibodies (e.g., bispecific antibodies). Among these, monoclonal antibodies are highly specific antibodies directed against a single antigenic site (epitope), and unlike polyclonal antibodies including different antibodies directed against different epitopes, monoclonal antibodies are directed only against a single epitope on an antigen, and thus quality control as a therapeutic agent is easy. The antibodies include variable region(s) of a heavy chain and/or a light chain of an immunoglobulin molecule, the variable region includes, as a primary structure thereof, a portion that forms an antigen-binding site of an antibody molecule, and the antibody of the present invention may be formed as some fragments containing the variable region. Preferably, the variable region may be replaced by a soluble receptor for CD300c, but is not limited thereto as long as it exhibits the same effect as that of an anti-CD300c antibody.

In the present specification, "prevention" means all actions that inhibit diseases such as cancer or delay the onset thereof via administration of the pharmaceutical composition according to the present invention.

In the present specification, "treatment" means all actions that alleviate or beneficially change symptoms due to cancer and the like via administration of the pharmaceutical composition according to the present invention.

In the present specification, "individual" refers to a subject to which the pharmaceutical composition of the present invention can be administered, and the subject is not limited.

In the present specification, "pharmaceutical composition" may be in the form of capsules, tablets, granules, an injection, an ointment, powders, or a beverage, and the pharmaceutical composition may be used for humans. The pharmaceutical composition is not limited to the above examples, and may be formulated in the form of oral preparations such as powder, granules, capsules, tablets, an aqueous suspension, and the like, preparations for external application, suppositories, and sterile injection solutions, according to general methods. The pharmaceutical composition of the present disclosure may include a pharmaceutically acceptable carrier. The pharmaceutically acceptable carrier may be a binder, a lubricant, a disintegrant, an excipient, a solubilizing agent, a dispersant, a stabilizer, a suspension agent, a pigment, a flavoring agent, or the like in the case of oral administration, may be used in combination with a buffer, a preservative, an analgesic agent, a solubilizer, an isotonic agent, a stabilizer, or the like in the case of injections, and may be a base, an excipient, a lubricant, a preservative, or the like in the case of local administration. Formulations of the pharmaceutical composition of the present disclosure may be formulated in a variety of ways by mixing with the above-described pharmaceutically acceptable carrier(s). For example, preparations for oral administration may be formulated in the form of tablets, troches, capsules, elixirs, suspensions, syrups, wafers, or the like, and preparations for injections may be formulated in unit dosage ampoules or in multiple dosage form. In addition, preparations of the pharmaceutical composition may be formulated in the form of solutions, suspensions, tablets, capsules, sustained release type preparations, or the like.

Meanwhile, examples of suitable carriers, excipients and diluents for formulation include lactose, dextrose, sucrose, sorbitol, mannitol, xylitol, erythritol, maltitol, starch, acacia gum, alginates, gelatin, calcium phosphate, calcium silicate, cellulose, methyl cellulose, micro-crystalline cellulose, polyvinylpyrrolidone, water, methylhydroxy benzoate, propylhydroxy benzoate, talc, magnesium stearate, mineral oil, and the like. In addition, the pharmaceutical composition may further include a filler, an anti-coagulant, a lubricant, a wetting agent, a flavoring agent, an emulsifier, a preservative, or the like.

Administration routes of the pharmaceutical composition according to the present disclosure include, but are not limited to, oral administration, intravenous administration, intramuscular administration, intraarterial administration, intramedullary administration, intradural administration, intracardiac administration, transdermal administration, subcutaneous administration, intraperitoneal administration, intranasal administration, intestinal administration, topical administration, sublingual administration, and rectal administration. Oral or parenteral administration is preferable. The term "parenteral" as used herein is intended to include subcutaneous, intradermal, intravenous, intramuscular, intraarticular, intrabursal, intrasternal, intradural, intralesional, and intracranial injections or injection techniques. The pharmaceutical composition of the present disclosure may also be administered in the form of a suppository for rectal administration.

The pharmaceutical composition of the present invention may vary depending on various factors including the activity of the used specific compound, age, body weight, general health, gender, diet, administration time, administration route, excretion rate, drug combination, and the severity of a particular disease to be prevented or treated, and a dosage of the pharmaceutical composition varies according to the condition and body weight of a patient, the severity of disease, drug form, administration route, and administration period, but may be appropriately selected by one of ordinary skill in the art, and may range from 0.0001 mg/kg/day to about 500 mg/kg/day or 0.001 mg/kg/day to 500 mg/kg/day. The pharmaceutical composition may be administered once or multiple times a day. The dosage is not intended to limit the scope of the present disclosure in any way. The pharmaceutical composition according to the present invention may be formulated into pills, dragees, capsules, a liquid, a gel, a syrup, a slurry, or a suspension.

Hereinafter, the following examples will be described to aid in understanding the present invention. However, these examples are provided merely to facilitate understanding of the present invention and are not intended to limit the scope of the present invention.

### Examples

### Example 1: Production of sCD300c-Fc

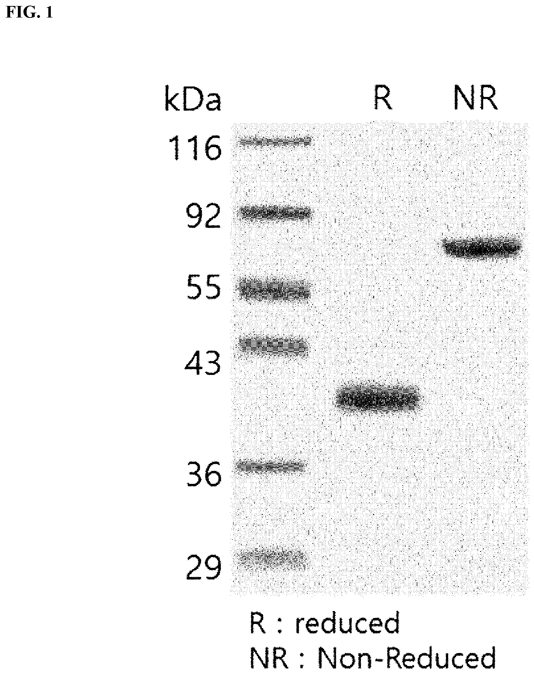

In order to analyze the function of CD300c in cancer cells and the immune system, sCD300c-Fc in which the Fc portion of a heavy chain region of an antibody was bound to soluble CD300c was produced. For the production of sCD300c-Fc, a gene (SEQ ID NO: 4) encoding the amino acid sequence of SEQ ID NO: 3 was inserted into pcDNA3.1 and transformed into a HEK293T cell line. In addition, to produce sCD300c-Fc, transformed cells and polymers that increase intracellular gene transfer efficiency were added to an RPMI medium supplemented with fetal bovine serum having an ultra-low IgG content and cultured in a cell incubator for 4 days. After the culture was completed, the supernatant containing sCD300c-Fc was separated using a centrifuge, and filtered once using a 0.22 µm filter. In addition, sCD300c-Fc was separated and purified using a recombinant protein-A Sepharose column (GE Healthcare), and the concentration of purified sCD300c-Fc was determined by measuring absorbance. Then, 2 µg of the purified sCD300c-Fc was added to a reducing sample buffer and a non-reducing sample buffer, respectively, and then electrophoresis was performed using pre-made SDS-PAGE gel (Invitrogen). Thereafter, the protein was stained using Coomassie Blue. The results thereof are shown in FIG. 1.

As illustrated in FIG. 1, it was confirmed that sCD300c-Fc having a purity of 90% or higher was purified.

### Example 2: Confirmation of Effect of sCD300c-Fc on Tumor-infiltrating Lymphocytes

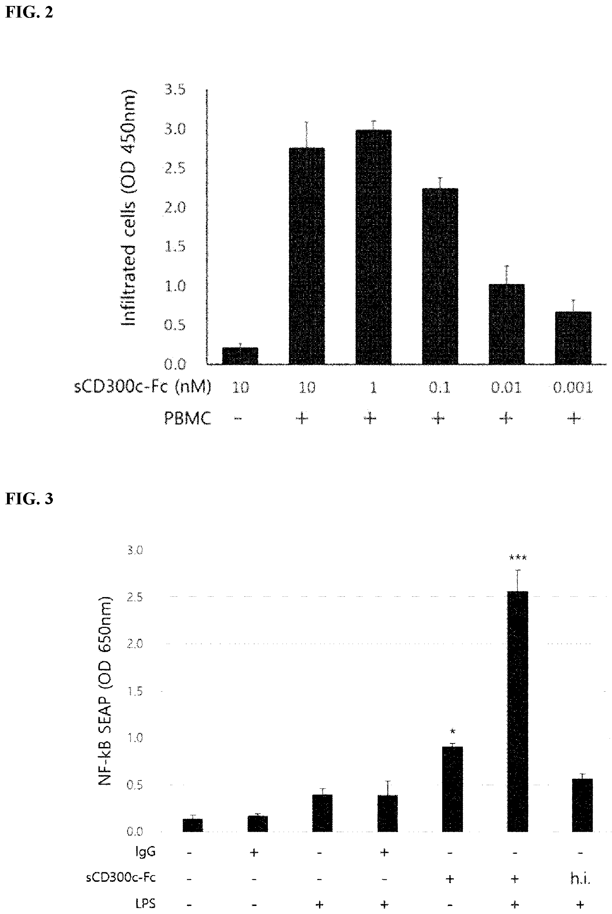

To confirm the effect of sCD300c-Fc on tumor-infiltrating lymphocytes (TILs), an experiment was conducted using sCD300c-Fc prepared in the same manner as in Example 1. More specifically, an EBM medium (endothelial basal medium, Lonza) supplemented with 1% fetal bovine serum (FBS) was dispensed into a 6-well plate, and 1 x 10⁶ cells/mL of human umbilical vein endothelial cells (HUVECs, PromoCell) and 1 x 10⁵ cells/mL of peripheral blood mononuclear cells (PBMCs, CTL) were inoculated and treated with sCD300c-Fc at concentrations of 10 nM, 1 nM, 0.1 nM, 0.01 nM, and 0.001 nM. Then, after culturing for 16 hours under conditions of 37 °C and 5% CO₂, absorbance at OD 450 nm was measured to measure tumor-infiltrating lymphocytes. The results thereof are shown in FIG. 2.

As illustrated in FIG. 2, it was confirmed that the higher the concentration of sCD300c-Fc, the greater the number of tumor-infiltrating lymphocytes, from which it was confirmed that, through the treatment of sCD300c-Fc, peripheral blood mononuclear cells were differentiated, resulting in an increase in the number of lymphocytes that infiltrated the human umbilical vein endothelial cells, and the number of lymphocytes was increased depending on the concentration of sCD300c-Fc. Through the above results, it was confirmed that immune cells can be activated through treatment with sCD300c-Fc.

### Example 3: Confirmation of Effect of sCD300c-Fc on Signaling Mechanism of NF-κB

In order to confirm the effect of sCD300c-Fc on the signaling of NF-κB, an experiment was conducted using sCD300c-Fc prepared in the same manner as in Example 1. More specifically, THP-1 blue cells (monocyte cells, InvivoGen) were inoculated into a 96-well plate at a concentration of 5,000 cells per well and cultured for 12 hours to stabilize the cells. Then, each well was treated with sCD300c-Fc, lipopolysaccharide (LPS), and/or IgG, followed by incubation at 37 °C and 5% CO₂ for 48 hours. Then, the culture supernatant was separated, treated with an SEAP coloring reagent (InvivoGen), and reacted for 1 hour, and then absorbance at 650 nm was measured to measure the signaling of NF-κB. The results thereof are shown in FIG. 3.

As illustrated in FIG. 3, it was confirmed that the NF-κB signal was about 0.4 in the control treated with only LPS, and the default value of 0.8 was shown in the control treated with sCD300c-Fc. In contrast, it was confirmed that, in the experimental group treated with both LPS and sCD300c-Fc, the signal of NF-κB was significantly increased. It was also confirmed that sCD300c-Fc (h.i. sCD300c-Fc; heat inactivated sCD300c-Fc) that was inactivated by heating did not affect the signal of NF-κB. Through the above results, it was confirmed that sCD300c-Fc activates the signaling mechanism of NF-κB, thereby activating THP-1 monocytes, which are immune cells, and promotes differentiation into macrophages, thereby effectively activating the innate immune system.

### Example 4: Production of Anti-CD300c Antibody

### 4.1. Production of Anti-CD300c Polyclonal Antibody

In order to produce a polyclonal antibody against CD300c, a gene (SEQ ID NO: 2) of the extracellular domain of CD300c to be used as an antigen was inserted into a pET28a (Novagen) expression vector to express 6x histidine, and transformed into *E. coli.* Then, the transformed *E. coli* was inoculated into 100 mL of an LB medium supplemented with 100 mg/mL of ampicillin, and then incubated so that the absorbance at OD 600 nm was 0.8-1.0, and IPTG was added. Then, after incubation at 25 °C for 16 hours to induce the expression of CD300c having a His-tag, the culture medium was centrifuged to remove the supernatant and cells were obtained. The obtained cells were resuspended using a solution in which 50 mM NaH₂PO₄ and 500 mM NaCl (pH 8.0) were mixed, and the cells were disrupted using ultrasound. Then, a cell lysate to be used for protein purification was obtained through centrifugation and filtration processes. CD300c with a His-tag contained in the cell lysate (SEQ ID NO: 5; CD300c-His) was purified using an affinity chromatography method using a Ni-NTA Sepharose column (GE Healthcare), and CD300c with a Hig-tag was eluted through step gradient elution. Purified CD300c having a His-tag was subjected to SDS-PAGE in the same manner as in Example 1, and it was confirmed that CD300c with a His-tag having a purity of 90% or higher was purified.

Then, antigen immunization was performed on New Zealand white rabbits using the purified CD300c with a His-tag. More specifically, the purified CD300c antigen with a Hig-tag was diluted with phosphate buffered saline (PBS) at a concentration of 0.5 mg/400 µL, mixed with the same amount of a complete Freund's adjuvant (Sigma), and subcutaneously injected, to perform primary immunization. After 2 weeks, 0.5 mg/400 µL of the antigen and the same amount of an incomplete Freund's adjuvant (Sigma) were mixed and used to perform secondary immunization using the same method, and then quaternary immunization was finally performed using the same method at intervals of 2 weeks. After tertiary immunization, blood was collected by collecting blood from the tail vein, and the obtained blood was diluted 1/1,000 to evaluate the activity of the antibody by ELISA. After the final quaternary immunization, whole blood was collected from the rabbits after anesthesia to obtain plasma.

### 4.2. Confirmation of Antibody Specificity of Anti-CD300c Antibody

In order to confirm the specificity of the anti-CD300c antibody produced in the same manner as in Example 4.1, the specificity for the CD300c antigen diluted at concentrations of 1 ng, 10 ng, 100 ng, 500 ng, and 1 µg was evaluated using plasma diluted 1,000-fold by ELISA and western blotting. As a result, it was confirmed that the anti-CD300c antibody specifically responded to the CD300c antigen.

### 4.3. Purification of Anti-CD300c Antibody

In order to purify the anti-CD300c antibody from plasma obtained in the same manner as in Example 4.1, an affinity purification method was used. More specifically, the CD300c antigen and NHS activated Sepharose Fast Flow resin (GE Healthcare) were mixed together using a coupling buffer (0.2 M NaHCO₃, 0.5 M NaCl, pH 8.3) to prepare an affinity gel, and then packed in a polypropylene column. Then, the plasma obtained in the column was added to leave only an antibody specifically binding to the antigen in the column, and then the antibody was purified using an elution buffer in which 0.1 M glycine (pH 2.5) and 0.1 M citric acid (pH 3.0) were mixed. Then, the purified antibody was dialyzed using phosphate buffered saline, concentrated, dispensed at a concentration of 1.0 mg/mL, and stored at -80 °C before use.

### Example 5: Confirmation of Effect of Anti-CD300c Antibody on T cells

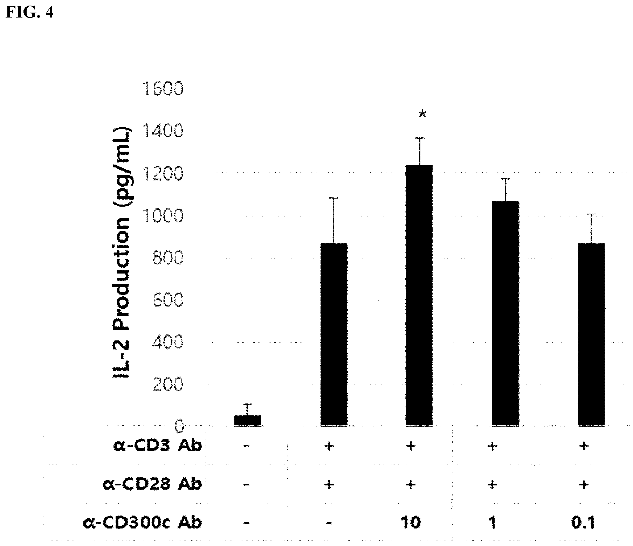

An experiment was conducted to confirm the effect of the anti-CD300c antibody on human T cells. More specifically, pan T cells were first isolated from human peripheral blood mononuclear cells (PBMCs) using a T cell separation kit (130-096-534, Milltenyi). Then, the isolated T cells were inoculated into a 96-well plate at a concentration of 5,000 cells per well, and cultured for 6 hours to stabilize the cells, and treated with an anti-CD3 antibody (Biolegend) and an anti-CD28 antibody (Biolegend). Then, the anti-CD300c polyclonal antibody purified in the same manner as in Example 4.3 was treated at various concentrations. After incubation at 37 °C for 48 hours, the culture supernatant was separated to measure the amount of IL-2. The amount of IL-2 was confirmed using an IL-2 Quantikine kit (R&D Systems). The results thereof are shown in FIG. 4.

As illustrated in FIG. 4, it was confirmed that, in the case of the experimental group treated with the anti-CD300c antibody, the amount of IL-2 increased according to the treatment concentration. Through this, it was confirmed that, in the case of the anti-CD300c antibody, the secretion of IL-2 was increased to activate T cells, which is an adaptive immune system.

### Example 6: Confirmation of In Vitro Anticancer Effect of Anti-CD300c Antibody

### 6.1. Confirmation of Effect of Anti-CD300c Antibody on Lung Cancer Growth

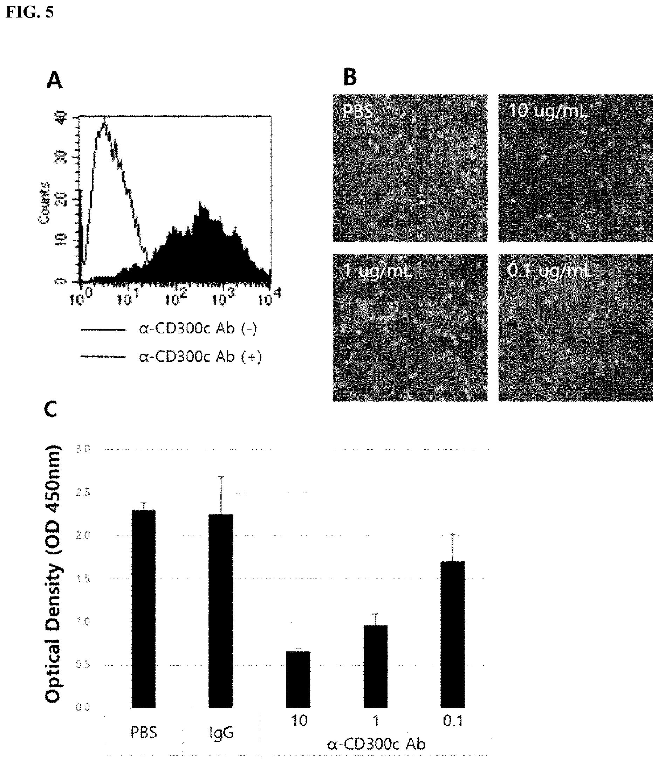

In order to confirm whether the anti-CD300c antibody has the effect of inhibiting lung cancer growth, it was first checked whether there is the CD300c antigen on the surface of A549 cells. More specifically, A549 cells, which are a human lung cancer cell line, were fixed with 4% formaldehyde and then blocked using 5% normal goat serum. Then, 1 µg of anti-CD300c antibody was treated and reacted, followed by staining with FITC-labeled anti-rabbit IgG antibodies. Then, the fluorescently labeled cells were confirmed using a flow cytometer (FACS). The results thereof are shown in FIG. 5A.

As illustrated in FIG. 5A, it was confirmed that the human lung cancer cell line had the CD300c antigen.

In addition, in order to confirm whether the anti-CD300c antibody inhibits the growth of lung cancer, a cancer cell proliferation inhibitory effect was confirmed. More specifically, A549 cells were inoculated into a 96-well plate at a concentration of 10,000 cells per well, followed by incubation for 18 hours to stabilize the cells. Then, the cells were treated with 0.1 µg/mL, 1 µg/mL, and 10 µg/mL of the anti-CD300c antibody and cultured for 72 hours. Then, images were captured using a microscope. The results thereof are shown in FIG. 5B. In addition, 10 µL of CCK-8 (Dijindo) was added to each well and allowed to react for 4 hours, and then absorbance at 450 nm was measured. The results thereof are shown in FIG. 5C.

As illustrated in FIGS. 5B and 5C, it was confirmed that, as the concentration of the treated anti-CD300c antibody increased, the proliferation of lung cancer cells was inhibited, from which it was confirmed that the anti-CD300c antibody inhibited the growth of lung cancer.

### 6.2. Confirmation of Inhibitory Effect of Anti-CD300c Antibody on Breast Cancer Growth

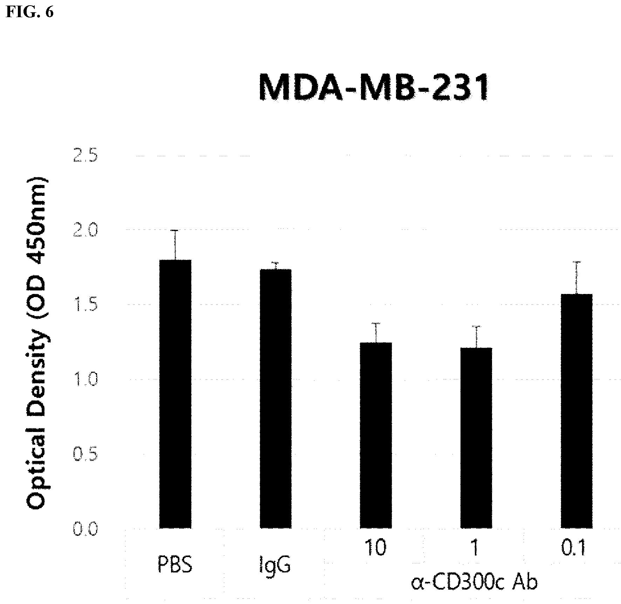

In order to confirm whether the anti-CD300c antibody has the effect of inhibiting the growth of breast cancer, a cell proliferation inhibitory effect was examined using MDA-MB-231 cells, which are a breast cancer cell line, in the same manner as in Example 6.1. The results thereof are shown in FIG. 6.

As illustrated in FIG. 6, it was confirmed that, as the concentration of the treated anti-CD300c antibody increased, the proliferation of breast cancer cells was inhibited, from which it was confirmed that the anti-CD300c antibody inhibited the growth of breast cancer.

### 6.3. Confirmation of Inhibitory Effect of Anti-CD300c Antibody on Colorectal Cancer Growth

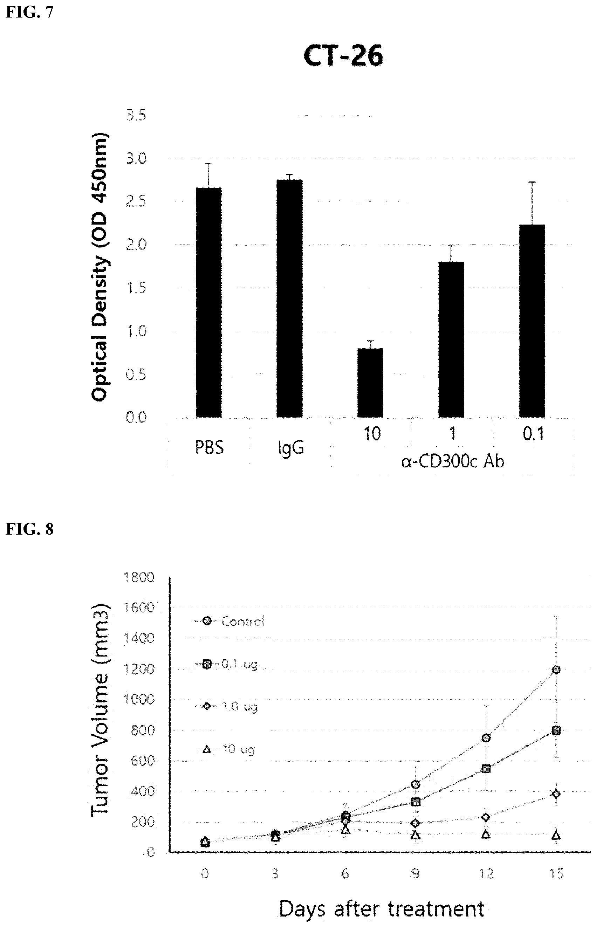

In order to confirm whether the anti-CD300c antibody has the effect of inhibiting the growth of colorectal cancer, a cell proliferation inhibitory effect was examined using CT-26 cells, which are a metastatic colorectal cancer cell line, in the same manner as in Example 6.1. The results thereof are shown in FIG. 7.

As illustrated in FIG. 7, it was confirmed that, as the concentration of the treated anti-CD300c antibody increased, the proliferation of colorectal cancer cells was inhibited, from which it was confirmed that the anti-CD300c antibody inhibited the growth of colorectal cancer.

### Example 7: Confirmation of In Vivo Anti-cancer Effect of Anti-CD300c Antibody

### 7.1. Confirmation of Inhibitory Effect of Anti-CD300c Antibody on Colorectal Cancer Growth

To confirm whether the anti-CD300c antibody has the effect of inhibiting the growth of cancer cells *in vivo,* CT-26 cells, which are a metastatic colorectal cancer cell line, were subcutaneously injected at a concentration of 5 x 10⁵ cells into the right side of 8-week-old BALB/c mice and the mice were raised with feed and water. Animal breeding and all experimental procedures were conducted in accordance with the laws and regulations for animal experiments. Then, when the tumor size reached about 70 mm³, the anti-CD300c antibody was intraperitoneally injected at concentrations of 0.1 µg, 1 µg, and 10 µg on day 0, day 1, and day 5, and then the tumor size was confirmed. As a control, phosphate buffered saline was injected. The results thereof are shown in FIG. 8.

As illustrated in FIG. 8, it was confirmed that, as the concentration of the anti-CD300c antibody increased, an increase in the size of metastatic colorectal cancer was reduced, and in the experimental group treated with 10 µg, tumor growth was completely inhibited until 15 days so that tumor growth no longer occurred.

### 7.2. Confirmation of Inhibitory Effect of Anti-CD300c Antibody on Lung Cancer Growth

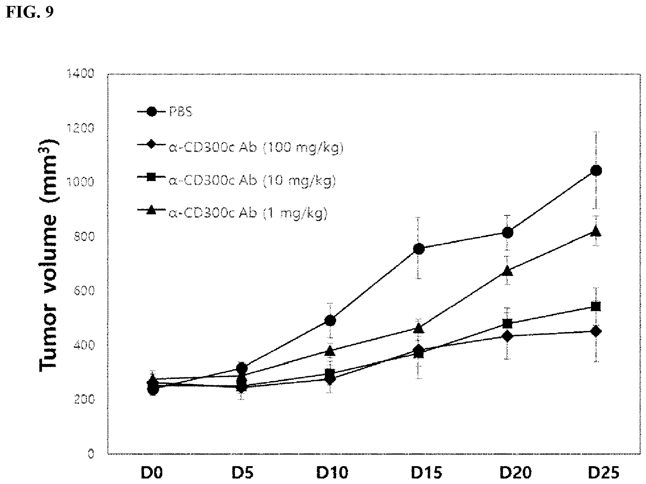

The same experiment as in Example 7.1 was performed using a lung cancer animal model. More specifically, A549 cells, which are a human lung cancer cell line, were subcutaneously injected at a concentration of 5 x 10⁶ cells into the right axilla of 4- to 6-week-old BALB/c mice, and the mice were raised with feed and water. Then, when the tumor diameter reached about 3 mm to about 5 mm, the anti-CD300c antibody was intraperitoneally injected at concentrations of 1 mg/kg, 10 mg/kg, and 100 mg/kg on day 0, day 1, and day 5, and then tumor size was confirmed. As a control, phosphate buffered saline was injected. The results thereof are shown in FIG. 9.

As illustrated in FIG. 9, it was confirmed that, as the concentration of the anti-CD300c antibody increased, the proliferation of lung cancer was inhibited and the tumor did not grow. Through the above results, it was confirmed that the anti-CD300c antibody of the present invention effectively inhibited the growth of cancer even *in vivo.*

Through the above results, it was confirmed that the anti-CD300c antibody can effectively inhibit the proliferation of various cancers, and that the anti-CD300c antibody can be used as an anticancer agent.

### Example 8: Confirmation of Anticancer Effect of CD300c siRNA

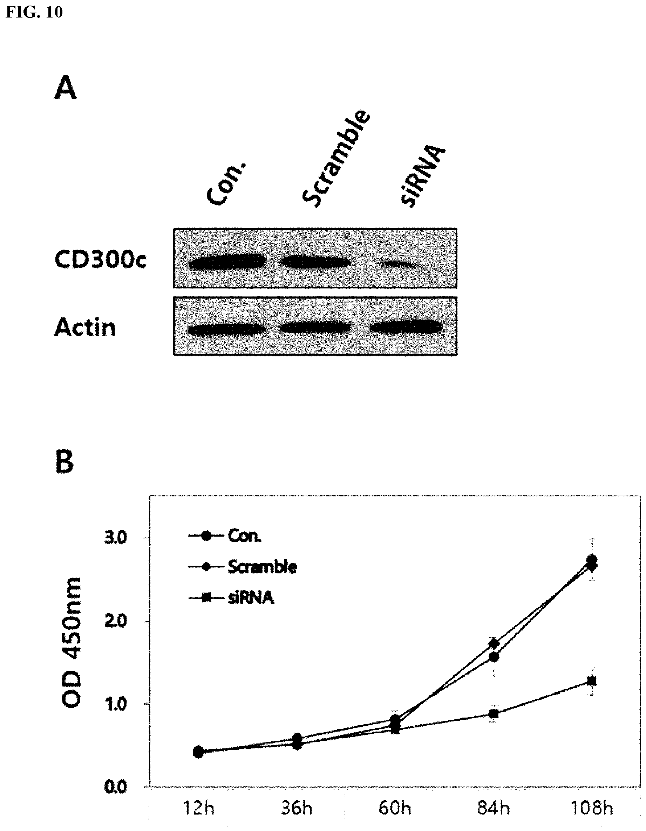

In order to further confirm the effect of CD300c on cancer cell proliferation, it was examined whether the inhibition of CD300c expression exhibited an anticancer effect. More specifically, the expression of CD300c was suppressed in A549 cells, which are a lung cancer cell line, in accordance with the manual provided using siRNA against CD300c (sc-93646, Santa Cruz). Scrambled RNA was used as a control. siRNA was transfected into cells using Lipofectamine RNAiMax (Life Technologies), and then cultured for 30 hours. Then, the cultured cells were treated with a cell lysis solution (20 mM Tris-HCl (pH 7.5), 150 mM NaCl, 1 mM Na₂EDTA, 1 mM EGTA, 1% Triton, 2.5 mM sodium pyrophosphate, 1 mM glycerophosphate, 1 mM Na₃VO₄, 1 µg/mL of leupeptin, and 1 mM PMSF) to recover a cell lysate. Then, the amount of protein contained in the lysate was quantified using a BCA method. Then, western blotting was performed using an antibody (PA5-87097, Thermo Fisher) specifically binding to the same amount of CD300c. The results thereof are shown in FIG. 10A. In addition, in order to confirm a cancer cell proliferation inhibitory effect, A549 cells were inoculated into a 96-well plate at a concentration of 10,000 cells/well, and then cultured for 18 hours to stabilize the cells. In addition, after treatment with siRNA, the cells were cultured for 5 days and absorbance was measured using CCK-8. The results thereof are shown in FIG. 10B.

As illustrated in FIG. 10A, it was confirmed that the expression of CD300c in the cells can be suppressed using CD300c siRNA. As illustrated in FIG. 10B, it was also confirmed that, when a cancer cell line was treated with siRNA, cell proliferation was inhibited.

Through the above results, it was confirmed that the anticancer effect can be exhibited by inhibiting the expression of CD300c, and it was also confirmed that, by inhibiting the expression of CD300c using small interfering RNA (siRNA), an antisense oligonucleotide (ASO), micro RNA (miRNA), or the like, or by inhibiting the activity of CD300a using an antibody, aptamer, or the like that specifically binds to CD300c, the anticancer effect can be exhibited in various cancers.

### Example 9: Confirmation of Anticancer Immune Effect through Inhibition of Function and/or Expression of CD300c

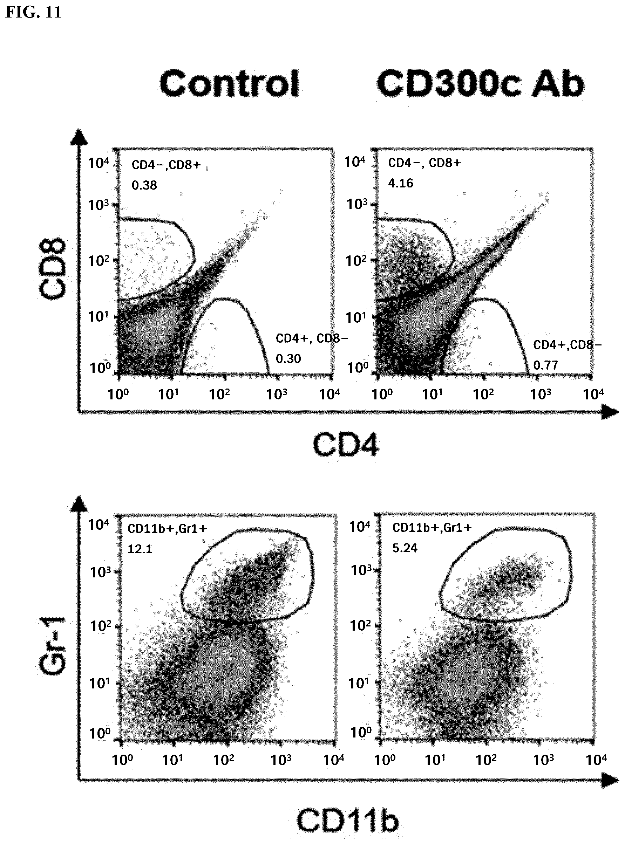

In order to confirm whether the anticancer immune effect is exhibited by inhibiting the function and/or expression of CD300c, 10 µg of the anti-CD300c antibody was intraperitoneally injected into BALB/c mice into which the colorectal cancer cell line was transplanted in the same manner as in Example 7.1, on day 0, day 1, and day 5, and after euthanasia on day 7, bone marrow was isolated from each mouse. Then, myeloid-derived suppressor cells (MDSCs), lymphocytes, and cytotoxic T lymphocytes from the isolated bone marrow were confirmed using a flow cytometer (FACS). The results thereof are shown in FIG. 11.

As illustrated in FIG. 11, it was confirmed that the number of lymphocytes and cytotoxic T lymphocytes (CD8+) were increased in the case of mice treated with the anti-CD300c antibody, and the differentiation induction (CD4+) of the lymphocytes was promoted. In contrast, it was confirmed that the number of myeloid-derived suppressor cells was reduced. Through the above results, it was confirmed that, by inhibiting the function and/or expression of CD300c, the activation of the immune system in the blood of the cancer animal model was induced, and by reducing the number of myeloid-derived suppressor cells that inhibit the activity or proliferation of T immune cells, the anticancer immune effect was remarkably increased, thus exhibiting a cancer treatment effect.

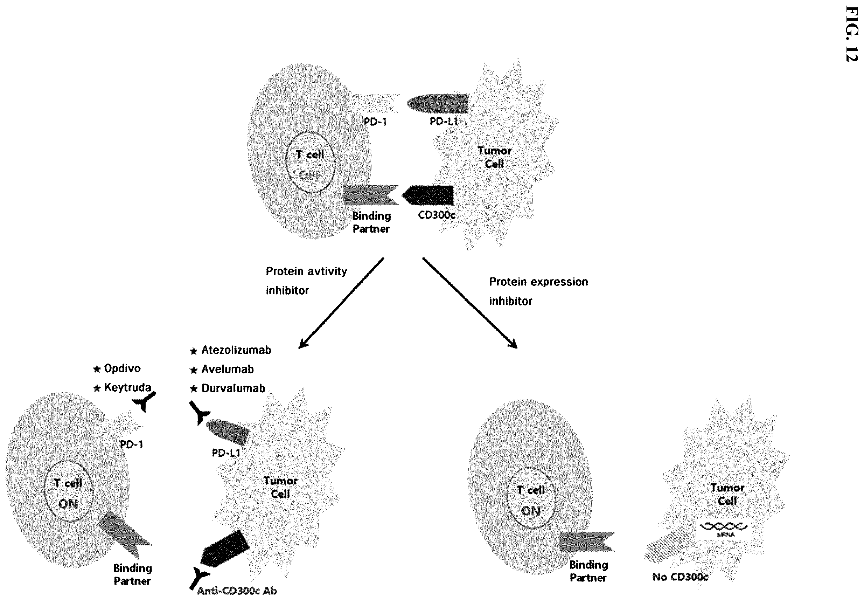

FIG. 12 is a schematic view illustrating mechanism of an anticancer effect by inhibiting the activity and/or expression of CD300c. CD300c, which is expressed on the surface of tumors, such as the B7 family (e.g., PD-1/PD-L1 interaction) that forms a known immune checkpoint serves to inhibit the activation of T cells by binding to a binding partner on the surface of T cells, but it is confirmed that, through treatment with an anti-CD300c antibody specifically binding to CD300c, the activity and proliferation of T cells are stimulated, thus exhibiting an anticancer effect, and at the same time, the anti-CD300c antibody binds to CD300c on the surface of tumor cells, thereby directly inhibiting tumor proliferation. It is confirmed that, even when the expression of CD300c on the surface of a tumor is inhibited using an oligonucleotide that inhibits CD300c expression, the same effect is exhibited.

Accordingly, since it was confirmed through the above results that, by inhibiting the expression and/or function of CD300c, the number of tumor-infiltrating lymphocytes and cytotoxic T lymphocytes in a cancer environment was increased, and the number of myeloid-derived suppressor cells was reduced, thereby effectively activating an anticancer immune response in the body, and the growth and development of cancer can also be effectively inhibited by suppressing cell proliferation, a material for inhibiting the expression and/or function of CD300c can be effectively used in inhibiting the proliferation, recurrence, metastasis, and the like of various cancers.

The above description of the present invention is provided only for illustrative purposes, and it will be understood by one of ordinary skill in the art to which the present invention pertains that the invention may be easily modified into other specific forms without departing from the technical spirit or essential characteristics thereof. Thus, the embodiments described herein should be considered in an illustrative sense only and not for the purpose of limitation.

### INDUSTRIAL APPLICABILITY

The present invention relates to a novel use of the CD300c protein present on the surface of various cancer cells, and it has been confirmed that, by inhibiting the expression or activity of the CD300c protein, the activity of T cells can be increased, and the proliferation of cancer cells can be inhibited. Thus, a CD300c expression inhibitor or activity inhibitor of the present invention not only can be applied to various cancers, but can also remarkably increase the effect of preventing and/or treating cancer, and accordingly, is expected to be applied to various cancer therapeutic agents and widely used.

## Claims

1. A pharmaceutical composition for preventing or treating cancer, the pharmaceutical composition comprising, as an active ingredient, a CD300c expression inhibitor or activity inhibitor.

2. The pharmaceutical composition of claim 1, wherein the CD300c expression inhibitor comprises any one or more selected from the group consisting of an antisense oligonucleotide (ASO), small hairpin RNA, small interfering RNA (siRNA), and a ribozyme that complementarily bind to mRNA of a CD300c gene.

3. The pharmaceutical composition of claim 1, wherein the CD300c activity inhibitor comprises any one or more selected from the group consisting of a compound, a peptide, a peptide mimetic, a substrate analog, an aptamer, and an antibody that complementarily bind to a CD300c protein.

4. The pharmaceutical composition of claim 1, wherein the cancer comprises any one or more selected from the group consisting of colorectal cancer, rectal cancer, colon cancer, thyroid cancer, oral cancer, pharyngeal cancer, laryngeal cancer, cervical cancer, brain cancer, lung cancer, ovarian cancer, bladder cancer, kidney cancer, liver cancer, pancreatic cancer, prostate cancer, skin cancer, tongue cancer, breast cancer, uterine cancer, gastric cancer, bone cancer, and blood cancer.

5. The pharmaceutical composition of claim 1, further comprising other anticancer agents.

6. The pharmaceutical composition of claim 1, wherein the pharmaceutical composition inhibits the proliferation, survival, metastasis, and recurrence of cancer, or resistance to an anticancer agent.

7. A method of screening for a material for preventing or treating cancer, the method comprising:
(a) culturing a cancer cell expressing a CD300c protein;
(b) treating the cultured cancer cell with a candidate material;
(c) measuring a CD300c expression level of the cell treated with the candidate material; and
(d) selecting a candidate material that reduces the CD300c expression level.

8. A method of screening for a material for preventing or treating cancer, the method comprising:
(a) treating a CD300c protein with a candidate material; and
(b) selecting a candidate material bound to the CD300c protein.

9. A method of treating cancer, the method comprising administering, to an individual, a composition comprising, as an active ingredient, a CD300c expression inhibitor or activity inhibitor.

10. A use of a composition for preventing or treating cancer, the composition comprising, as an active ingredient, a CD300c expression inhibitor or activity inhibitor.


<110>    CentricsBio

<120>    Pharmaceutical composition comprising inhibitors of CD300c's
         expression or activation for preventing or treating of cancer

<130>    MPCT19-065

<150>    KR 10-2018-0062620
<151>    2018-05-31

<150>    KR 10-2019-0061067
<151>    2019-05-24

<160>    6

<170>    KoPatentIn 3.0

<210>    1
<211>    163
<212>    PRT
<213>    Artificial Sequence

<220>
<223>    extracellular domain of human CD300c


<400>    1
Gly Tyr Phe Pro Leu Ser His Pro Met Thr Val Ala Gly Pro Val Gly
  1               5                  10                  15

Gly Ser Leu Ser Val Gln Cys Arg Tyr Glu Lys Glu His Arg Thr Leu
             20                  25                  30

Asn Lys Phe Trp Cys Arg Pro Pro Gln Ile Leu Arg Cys Asp Lys Ile
         35                  40                  45

Val Glu Thr Lys Gly Ser Ala Gly Lys Arg Asn Gly Arg Val Ser Ile
     50                  55                  60

Arg Asp Ser Pro Ala Asn Leu Ser Phe Thr Val Thr Leu Glu Asn Leu
 65                  70                  75                  80

Thr Glu Glu Asp Ala Gly Thr Tyr Trp Cys Gly Val Asp Thr Pro Trp
                 85                  90                  95

Leu Arg Asp Phe His Asp Pro Ile Val Glu Val Glu Val Ser Val Phe
            100                 105                 110

Pro Ala Gly Thr Thr Thr Ala Ser Ser Pro Gln Ser Ser Met Gly Thr
        115                 120                 125

Ser Gly Pro Pro Thr Lys Leu Pro Val His Thr Trp Pro Ser Val Thr
    130                 135                 140

Arg Lys Asp Ser Pro Glu Pro Ser Pro His Pro Gly Ser Leu Phe Ser
145                 150                 155                 160

Asn Val Arg



<210>    2
<211>    489

<212>    DNA
<213>    Artificial Sequence

<220>
<223>    extracellular domain of human CD300c


<400>    2
ggctattttc ctctgagcca ccccatgacc gtggcgggcc ccgtgggggg atccctgagt        60

gtgcagtgtc gctatgagaa ggaacacagg accctcaaca aattctggtg cagaccacca       120

cagattctcc gatgtgacaa gattgtggag accaaagggt cagcagggaa aaggaatggc       180

cgagtgtcca tcagggacag tcctgcaaac ctcagcttca cagtgaccct ggagaatctc       240

acagaggagg acgcaggcac ctactggtgt ggggtggata caccgtggct ccgagacttt       300

catgatccca ttgtcgaggt tgaggtgtcc gtgttcccgg ccgggacgac cacagcctcc       360

agcccccaga gctccatggg cacctcaggt cctcccacga agctgcccgt gcacacctgg       420

cccagcgtga ccagaaagga cagccccgaa cccagcccac accctggctc cctgttcagc       480

aatgtccgc                                                               489


<210>    3
<211>    492
<212>    PRT
<213>    Artificial Sequence

<220>
<223>    CD300c-Fc


<400>    3
Gly Tyr Phe Pro Leu Ser His Pro Met Thr Val Ala Gly Pro Val Gly
1               5                   10                  15

Gly Ser Leu Ser Val Gln Cys Arg Tyr Glu Lys Glu His Arg Thr Leu
            20                  25                  30

Asn Lys Phe Trp Cys Arg Pro Pro Gln Ile Leu Arg Cys Asp Lys Ile
        35                  40                  45

Val Glu Thr Lys Gly Ser Ala Gly Lys Arg Asn Gly Arg Val Ser Ile
    50                  55                  60

Arg Asp Ser Pro Ala Asn Leu Ser Phe Thr Val Thr Leu Glu Asn Leu
65                  70                  75                  80

Thr Glu Glu Asp Ala Gly Thr Tyr Trp Cys Gly Val Asp Thr Pro Trp
                85                  90                  95

Leu Arg Asp Phe His Asp Pro Ile Val Glu Val Glu Val Ser Val Phe
            100                 105                 110

Pro Ala Gly Thr Thr Thr Ala Ser Ser Pro Gln Ser Ser Met Gly Thr
        115                 120                 125

Ser Gly Pro Pro Thr Lys Leu Pro Val His Thr Trp Pro Ser Val Thr
    130                 135                 140

Arg Lys Asp Ser Pro Glu Pro Ser Pro His Pro Gly Ser Leu Phe Ser
145                 150                 155                 160

Asn Val Arg Ser Thr Lys Gly Pro Ser Val Phe Pro Leu Ala Pro Ser
            165                 170                 175

Ser Lys Ser Thr Ser Gly Gly Thr Ala Ala Leu Gly Cys Leu Val Lys
        180                 185                 190

Asp Tyr Phe Pro Glu Pro Val Thr Val Ser Trp Asn Ser Gly Ala Leu
    195                 200                 205

Thr Ser Gly Val His Thr Phe Pro Ala Val Leu Gln Ser Ser Gly Leu
    210                 215                 220

Tyr Ser Leu Ser Ser Val Val Thr Val Pro Ser Ser Ser Leu Gly Thr
225                 230                 235                 240

Gln Thr Tyr Ile Cys Asn Val Asn His Lys Pro Ser Asn Thr Lys Val
            245                 250                 255

Asp Lys Lys Val Glu Pro Lys Ser Cys Asp Lys Thr His Thr Cys Pro
        260                 265                 270

Pro Cys Pro Ala Pro Glu Leu Leu Gly Gly Pro Ser Val Phe Leu Phe
    275                 280                 285

Pro Pro Lys Pro Lys Asp Thr Leu Met Ile Ser Arg Thr Pro Glu Val
    290                 295                 300

Thr Cys Val Val Val Asp Val Ser His Glu Asp Pro Glu Val Lys Phe
305                 310                 315                 320

Asn Trp Tyr Val Asp Gly Val Glu Val His Asn Ala Lys Thr Lys Pro
            325                 330                 335

Arg Glu Glu Gln Tyr Asn Ser Thr Tyr Arg Val Val Ser Val Leu Thr
        340                 345                 350

Val Leu His Gln Asp Trp Leu Asn Gly Lys Glu Tyr Lys Cys Lys Val
    355                 360                 365

Ser Asn Lys Ala Leu Pro Ala Pro Ile Glu Lys Thr Ile Ser Lys Ala
    370                 375                 380

Lys Gly Gln Pro Arg Glu Pro Gln Val Tyr Thr Leu Pro Pro Ser Arg
385                 390                 395                 400

Asp Glu Leu Thr Lys Asn Gln Val Ser Leu Thr Cys Leu Val Lys Gly
            405                 410                 415

Phe Tyr Pro Ser Asp Ile Ala Val Glu Trp Glu Ser Asn Gly Gln Pro
        420                 425                 430

Glu Asn Asn Tyr Lys Thr Thr Pro Pro Val Leu Asp Ser Asp Gly Ser
    435                 440                 445

Phe Phe Leu Tyr Ser Lys Leu Thr Val Asp Lys Ser Arg Trp Gln Gln
    450                 455                 460

Gly Asn Val Phe Ser Cys Ser Val Met His Glu Ala Leu His Asn His
465                 470                 475                 480

Tyr Thr Gln Lys Ser Leu Ser Leu Ser Pro Gly Lys
                485                 490

<210>    4
<211>    1478
<212>    DNA
<213>    Artificial Sequence

<220>
<223>    CD300c-Fc

<400>    4
ggctattttc ctctgagcca ccccatgacc gtggcgggcc ccgtgggggg atccctgagt      60

gtgcagtgtc gctatgagaa ggaacacagg accctcaaca aattctggtg cagaccacca     120

cagattctcc gatgtgacaa gattgtggag accaaagggt cagcagggaa aaggaatggc     180

cgagtgtcca tcagggacag tcctgcaaac ctcagcttca cagtgaccct ggagaatctc     240

acagaggagg acgcaggcac ctactggtgt ggggtggata caccgtggct ccgagacttt     300

catgatccca ttgtcgaggt tgaggtgtcc gtgttcccgg ccgggacgac cacagcctcc     360

agcccccaga gctccatggg cacctcaggt cctcccacga agctgcccgt gcacacctgg     420

cccagcgtga ccagaaagga cagccccgaa cccagcccac accctggctc cctgttcagc     480

aatgtccgcc ctccaccaag ggcccatcgg tcttccccct ggcaccctcc tccaagagca     540

cctctggggg cacagcggcc ctgggctgcc tggtcaagga ctacttcccc gaaccggtga     600

cggtgtcgtg gaactcaggc gccctgacca gcggcgtgca caccttcccg gctgtcctac     660

agtcctcagg actctactcc ctcagcagcg tggtgaccgt gccctccagc agcttgggca     720

cccagaccta catctgcaac gtgaatcaca agcccagcaa caccaaggtg gacaagaaag     780

ttgagcccaa atcttgtgac aaaactcaca catgcccacc gtgcccagca cctgaactcc     840

tggggggacc gtcagtcttc ctcttccccc caaaacccaa ggacaccctc atgatctccc     900

ggacccctga ggtcacatgc gtggtggtgg acgtgagcca cgaagaccct gaggtcaagt     960

tcaactggta cgtggacggc gtggaggtgc ataatgccaa gacaaagccg cgggaggagc    1020

agtacaacag cacgtaccgg gtggtcagcg tcctcaccgt cctgcaccag gactggctga    1080

atggcaagga gtacaagtgc aaggtctcca acaaagccct cccagccccc atcgagaaaa    1140

ccatctccaa agccaaaggg cagccccgag aaccacaggt gtacaccctg cccccatccc    1200

gggatgagct gaccaagaac caggtcagcc tgacctgcct ggtcaaaggc ttctatccca    1260

gcgacatcgc cgtggagtgg gagagcaatg ggcagccgga gaacaactac aagaccacgc    1320

ctcccgtgct ggactccgac ggctccttct tcctctacag caagctcacc gtggacaaga    1380

gcaggtggca gcaggggaac gtcttctcat gctccgtgat gcatgaggct ctgcacaacc    1440

actacacgca gaagagcctc tccctgtctc cgggtaaa                              1478

<210>    5
<211>    169
<212>    PRT
<213>    Artificial Sequence

<220>
<223>    CD300c-His

<400>    5
Gly Tyr Phe Pro Leu Ser His Pro Met Thr Val Ala Gly Pro Val Gly
1               5                   10                  15

Gly Ser Leu Ser Val Gln Cys Arg Tyr Glu Lys Glu His Arg Thr Leu
            20                  25                  30

Asn Lys Phe Trp Cys Arg Pro Pro Gln Ile Leu Arg Cys Asp Lys Ile
        35                  40                  45

Val Glu Thr Lys Gly Ser Ala Gly Lys Arg Asn Gly Arg Val Ser Ile
    50                  55                  60

Arg Asp Ser Pro Ala Asn Leu Ser Phe Thr Val Thr Leu Glu Asn Leu
65                  70                  75                  80

Thr Glu Glu Asp Ala Gly Thr Tyr Trp Cys Gly Val Asp Thr Pro Trp
                85                  90                  95

Leu Arg Asp Phe His Asp Pro Ile Val Glu Val Glu Val Ser Val Phe
            100                 105                 110

Pro Ala Gly Thr Thr Thr Ala Ser Ser Pro Gln Ser Ser Met Gly Thr
        115                 120                 125

Ser Gly Pro Pro Thr Lys Leu Pro Val His Thr Trp Pro Ser Val Thr
    130                 135                 140

Arg Lys Asp Ser Pro Glu Pro Ser Pro His Pro Gly Ser Leu Phe Ser
145                 150                 155                 160

Asn Val Arg His His His His His His
                165

<210>    6
<211>    507
<212>    DNA
<213>    Artificial Sequence

<220>
<223>    CD300c-His

<400>    6
ggctattttc ctctgagcca ccccatgacc gtggcgggcc ccgtgggggg atccctgagt      60

gtgcagtgtc gctatgagaa ggaacacagg accctcaaca aattctggtg cagaccacca     120

cagattctcc gatgtgacaa gattgtggag accaaagggt cagcagggaa aaggaatggc     180

cgagtgtcca tcagggacag tcctgcaaac ctcagcttca cagtgaccct ggagaatctc 240

acagaggagg acgcaggcac ctactggtgt ggggtggata caccgtggct ccgagacttt 300

catgatccca ttgtcgaggt tgaggtgtcc gtgttcccgg ccgggacgac cacagcctcc 360

agcccccaga gctccatggg cacctcaggt cctcccacga agctgcccgt gcacacctgg 420

cccagcgtga ccagaaagga cagccccgaa cccagcccac accctggctc cctgttcagc 480

aatgtccgcc atcatcatca tcatcac 507